# EUROPEAN PATENT SPECIFICATION

(11) **EP 1 356 791 B1**
(45) Date of publication and mention of the grant of the patent: **19.04.2006**
(21) Application number: 02256062.7
(22) Date of filing: 30.08.2002
(51) Int. Cl.: A61F 2/16, A61F 9/00

(54) **Intraocular lens insertion device**
Implantationsgerät für Intraokularlinse
Dispositif pour injection d'une lentille intraoculaire

(30) Priority: 01.04.2002 JP 2002098948
(43) Date of publication of application: 29.10.2003
(73) Proprietor: Canon-Staar Co., Inc., Tokyo (JP)
(72) Inventor: Kobayashi, Kenichi, Katsushika-ku, Tokyo (JP); Kikuchi, Toshikazu, Hachioji-shi, Tokyo (JP)
(74) Representative: Stanley, David William

(56) References cited:
- US-A- 4 173 281
- US-A- 4 715 373
- US-A- 5 190 552

## Description

The present invention relates to an insertion device for inserting an intraocular lens into the eye.

Implantation of an intraocular lens for treating a cataract has been widely performed since an artificial lens; i.e., an intraocular lens, was implanted for the first time into the human eye in place of an opaqued natural lens during cataract surgery. The applicant of the present application has proposed various types of intraocular lens insertion devices for implanting an intraocular lens into the lenticular capsule from which an opaqued natural lens has been removed, as disclosed in Japanese Patent Application Laid-Open (kokai) Nos. 4-212350, 5-103803, 5-103808, 5-103809, and 7-023990. By use of these tools, the optical portion of an intraocular lens is compressed, rolled, bent, stretched, or folded so as to reduce its external size, thereby making it possible to insert the intraocular lens into the eye through a small incision formed in the eyeball. These insertion tools facilitate an operation for reliably implanting an intraocular lens into the eye.

US 4715373 also discloses a device for inserting an intraocular lens into an eye.

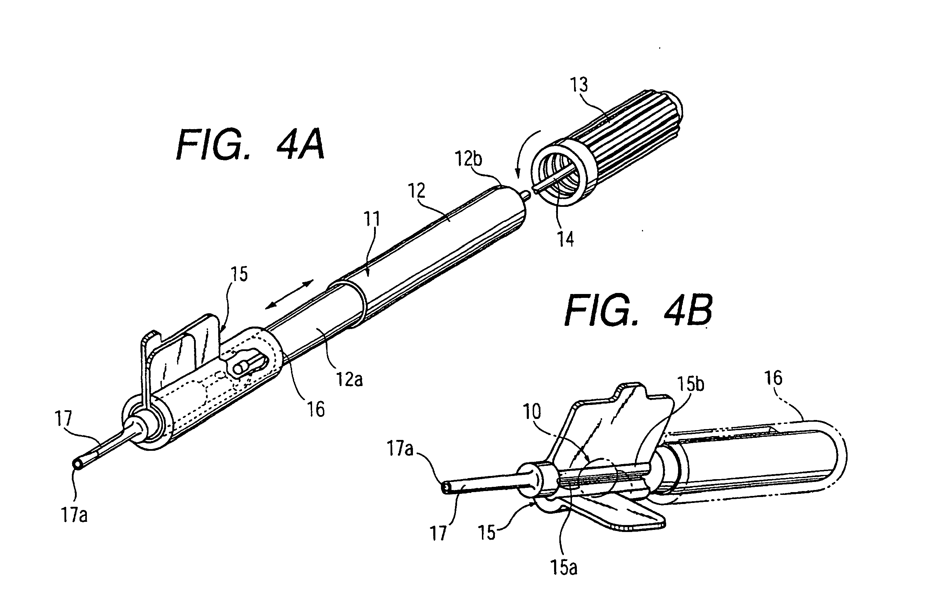

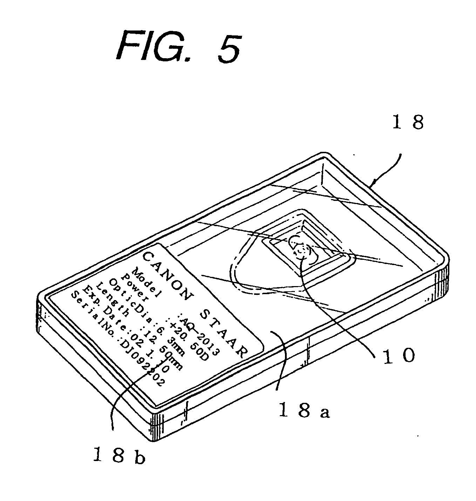

Reference will now be made to Figures 4 and 5 of the accompanying diagrammatic drawings, in which:
Figure 4A is a partially cutaway overall perspective view of a conventional insertion device for inserting a deformable intraocular lens;
Figure 4B is an enlarged perspective view of a main portion of the insertion device; and
Figure 5 is a perspective view of a conventional storage container for storing an intraocular lens.

In Figures 4A and 4B, reference numeral 11 denotes the insertion device for deforming an intraocular lens 10 to a smaller size and inserting the same into the eye; 12 denotes a hollow cylindrical device body; 13 denotes a screw sleeve; 14 denotes a push rod; 15 denotes an enclosing member having a lens receiving section 15a and an open/close mechanism 15b and adapted to deform the intraocular lens 10 to a smaller size; 16 denotes a slide stopper which engages the open/close mechanism 15b so as to maintain the same in a closed state; and 17 denotes an insertion tube.

When the insertion device 11 is used to insert the deformable intraocular lens 10 into the eye through a small incision, the open/close mechanism 15b of the enclosing member 15, which is connected to the front end 12a of the device body 12, is first opened. Subsequently, the deformable intraocular lens 10 is placed on the lens receiving section 15a of the enclosing member 15, and the open/close mechanism 15b of the enclosing member 15 is closed so as to reduce the exterior size of the deformable intraocular lens 10. Subsequently, the slide stopper 16 attached to the device body 12 is moved toward the lens receiving section 15a to engage the open/close mechanism 15b and bring the same into a closed state. Thus, placement of the intraocular lens 10 into the lens receiving section 15a is completed.

Subsequently, the screw sleeve 13, disposed at the rear of the device body 12, is moved toward the device body 12, brought into engagement with a screw portion 12b formed on the device body 12, and rotated in order to advance the push rod 14 in order to push forward the deformable intraocular lens 10 from the lens receiving section 15a. As a result, the deformable intraocular lens 10 is inserted into the eye through a tapered tip end 17a of an insertion tube 17 provided at the front end of the lens receiving section 15a, which tip end 17a has been inserted into the eye through a small incision formed in the eyeball. The operation of placing the intraocular lens 10 on the enclosing member 15 provided on the device body 12 is performed at the beginning of a surgical operation. Specifically, an intraocular lens 10 stored in an intraocular lens storage container 18 as shown in Figure 5 is selected, removed from the container 18, and placed on the enclosing member 15.

Since eyesight differs among users (patients) who require intraocular lenses 10, many types of intraocular lens 10 are provided.

Therefore, in order to enable identification of a required intraocular lens 10 among many types, as shown in Figure 5, lens data 18b are conventionally provided on a cover top face 18a of the storage container 18 storing an intraocular lens 10. The lens data include the name of a manufacturer of the lens, the model number of the lens, and other information. On the basis of the lens data, an operator selects a necessary intraocular lens 10, and sets the same onto the intraocular lens insertion device 11 for use at the time of an operation.

US 4173281 refers to the provision of lens data on a printed label affixed to a lens package.

When operations are performed concurrently on a large number of users (patients), in some cases, one assistant, such as a nurse, sets different types of intraocular lenses 10 having necessary optical characteristics onto different intraocular lens insertion devices 11 to be used for the patients, and hands the intraocular lens insertion devices to an operating surgeon. In such a case, the operating surgeon cannot confirm the type of the intraocular lens 10 before inserting the same into the eye of a patient, and may erroneously insert into the eye of the patient an intraocular lens having optical characteristics that are not suitable for the patient

Preferred embodiments of the present invention aim to solve the above-described problem involved in conventional intraocular lens insertion devices, and to provide an intraocular lens insertion device which can be manufactured at a plant in the form of a lens-containing insertion device loaded with an intraocular lens and which enables an user to know, from the outside of the insertion device, lens data, such as specifications and performance of the loaded intraocular lens.

Another aim is to provide an intraocular lens insertion device which enables an operating surgeon to confirm optical characteristics of an intraocular lens immediately before insertion thereof, even when an ordinary intraocular lens insertion device is used and the operating surgeon does not load the intraocular lens onto an intraocular lens insertion device (i.e. a nurse or assistant loads the intraocular lens into an intraocular lens insertion device).

According to one aspect of the present invention, there is provided an intraocular lens insertion device for inserting an intraocular lens into an eye, the device comprising data display means which displays data regarding a lens loaded into the intraocular lens insertion device, the data display means being provided at a predetermined visible position on an outer surface of the intraocular lens insertion device so as to enable the data regarding the loaded lens to be checked from the outside of the intraocular lens insertion device.

Embodiments of the present invention enable provision of an intraocular lens insertion device which can be manufactured at a plant in the form of a lens-containing insertion device loaded with an intraocular lens and which enables a user to know, from the outside of the insertion device, lens data, such as specifications and performance of an intraocular lens loaded onto the insertion device.

In addition, embodiments of the present invention can be applied to the case in which a conventional insertion device is used; i.e., the case in which an intraocular lens is removed from a lens storage container and placed on an insertion device immediately before an operation. In this case, a decal on which data of the intraocular lens is printed is stored in the lens storage container together with the intraocular lens; and simultaneously with loading of the intraocular lens into the insertion device, the decal is attached to a visible position of the outer surface of the device body. This enables an operating surgeon to confirm optical characteristics of an intraocular lens immediately before insertion thereof, even when the operating surgeon does not load the intraocular lens into the intraocular lens insertion device.

Moreover, such an insertion device enables an operator to check data of a lens loaded into the insertion device on the basis of the data display means provided on the insertion device. Therefore, through employment of a system in which two persons (e.g. a nurse and an operating surgeon) check optical characteristics of an intraocular lens to be used in an operation, use of a wrong intraocular lens can be prevented, and thus safety of operation can be secured.

Preferably, the data display means is a set of character data.

The data display means may be a change in colour provided at a predetermined location on a device body of the intraocular lens insertion device.

The data display means may be a string of characters engraved, by use of a laser, at an externally visible location on an outer surface of a device body of the intraocular lens insertion device.

The data display means may be a printed decal which carries character data and is visibly attached to an outer surface of a device body of the intraocular lens insertion device.

The data display means may be a string of characters or marks moulded at an externally visible position on a device body of the intraocular lens insertion device.

The data display means may be a string of characters printed directly on an outer surface of a device body of the intraocular lens insertion device.

In embodiments of the present invention, where character data are provided at a visible position on the outer surface of the insertion device by means of printing, engraving, or moulding, a large amount of information can be displayed clearly, and checking of the character data is facilitated.

In another aspect, the invention provides a method of preparing an intraocular lens for insertion, comprising the steps of loading the lens into an intraocular lens insertion device, and providing on the insertion device data display means displaying data regarding the loaded lens such that said data is visible and capable of being checked from the outside of the insertion device, prior to inserting the intraocular lens.

The intraocular lens insertion device is preferably in accordance with any of the preceding aspects of the invention.

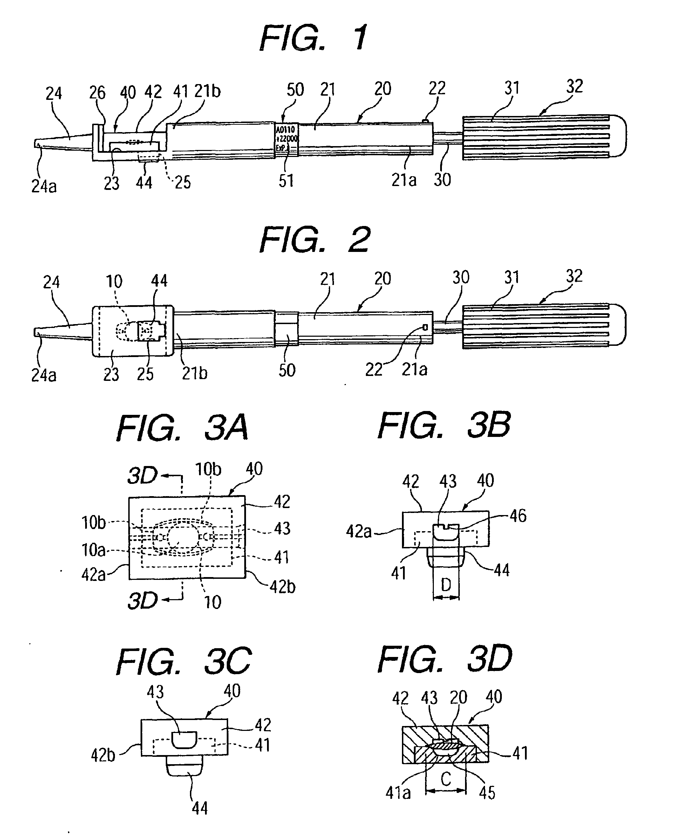

For a better understanding of the invention, and to show how embodiments of the same may be carried into effect, reference will now be made, by way of example, to Figures 1 to 3 of the accompanying diagrammatic drawings, in which:
Figure 1 is a front view of one example of an intraocular lens insertion device according to an embodiment of the present invention;
Figure 2 is a bottom view of the intraocular lens insertion device of Figure 1; and
Figures 3A to 3D are view showing a lens case wherein Figure 3A is a plan view of the lens case, Figure 3B is a left side view of the lens case, Figure 3C is a right side view of the lens case, and Figure 3D is cross-sectional view taken along line IIID-IIID of Figure 3A.

Figure 1 shows a state in which a lens case loaded with an intraocular lens is loaded into the insertion device 20.

In Figures 1 and 2, reference numeral 20 denotes an intraocular lens insertion device which includes a hollow cylindrical device body 21, a push rod 30, a pusher mechanism 32, an attachment section 23, and a tapered insertion tube 24 and is adapted to deform an intraocular lens 10 to a smaller size and push out the same from a tip end portion 24a of the insertion tube 24. The device body 21 is formed of transparent or semi-transparent plastic and has a base end portion 21 a of smaller diameter and a rip end portion 21 b of larger diameter. The push rod 30 is disposed to be located on the centre axis of the device body 21. The pusher mechanism 32 is constituted by a female screw sleeve 31, which is attached to a rear end portion of the push rod 30 and brought into screw-engagement with a male screw 22 formed on the base end portion 21a of the device body 21. With this configuration, the pusher mechanism 32 moves the push rod 30 back and forth upon rotation of the screw sleeve 31. The attachment portion 23 is formed on the tip end portion 21 b and adapted to receive a lens case 40. The insertion tube 24 is formed on the tip end side of the attachment portion 23 and has a through-hole aligned with the centre axis of the device body 21.

Reference numeral 25 denotes an engagement hole which is formed in the horizontal region of the attachment portion 23; and 26 denotes a notch which is formed on the rearward-facing surface of a front-side vertical wall of the attachment portion 23. The engagement hole 25 and the notch 26 cooperate to position the lens case 40 in relation to the attachment portion 23.

As is clearly shown in Figures 3A to 3D, the lens case 40 is constituted through assembly of a lens case top 41, on which the intraocular lens 10 is placed, and a lens case bottom 42 formed to cover the upper face of the intraocular lens 10 placed on the lens case top 41.

A groove-shaped depression 41 a is formed on the upper surface of the lens case top 41 such that the depression 41 a extends in the horizontal direction in Figure 3A. Support portions 10b of the intraocular lens 10 for supporting the optical portion 10a of the intraocular lens 10 are placed in the depression 41a. A projection 44 to be engaged with the engagement hole 25 of the attachment portion 23 is formed on the lower surface of the lens case top 41 to be located at a position which is offset slightly to the right (as seen) from the centre in Figure 3A.

The lens case bottom 42 for covering the upper surface of the lens case top 41 is formed such that the lens case bottom 42 can be fitted onto the lens case top 41 while covering the upper surface and circumferential (side) surface of the lens case top 41. A through hole 43 is formed in the left and right walls 42a and 42b of the lens case bottom 42 such that the centre axis of the through hole 43 is aligned with the centre axis of the device body 21 of the insertion device 20 when the lens case 40 is attached to the attachment portion 23.

The specific procedure for placement of the lens case 40 is as follows. First, the intraocular lens 10 is placed on the lens case top 41 such that the optical portion 10a of the lens 10 corresponds to the depression 41a of the lens case top 41 and the support portions 10b of the intraocular lens 10 are supported by the edge portions of the depression 41a. Subsequently, the lens case bottom 42 is placed on the lens case top 41, so that a space 45 is formed between the lens case bottom 42 and the lens case top 41. The space 45 has a shape and size such that movement of the intraocular lens 10 within the space 45 is restricted. Further, the shape of the space 45 is determined such that the optical portion 10a of the intraocular lens 10 does not come into contact with the inner surface of the lens case top 41 or the inner surface of the lens case bottom 42, thereby preventing the optical characteristics of the optical portion 10a from changing due to force acting on the optical portion 10a during long-term storage.

The through hole 43 of the lens case 40 is reduced in diameter toward the tip end thereof. The intraocular lens 10 and the push rod 30 pass through the through hole 43. After attachment of the lens case 40 to the insertion device 20, the push rod 30 is advanced through operation of the pusher mechanism 32. As a result, the intraocular lens 10 within the lens case 40 is gradually deformed to a smaller size and moved into the insertion tube 24. Subsequently, after the tip end 24a of the insertion tube 24 is inserted into the eye through an incision formed therein, the push rod 30 is advanced further, so that the intraocular lens 10 is deformed to a further reduced size by means of the insertion tube 24 and then pushed into the eye.

Figures 3B, 3C, and 3D show the diameter of the through hole 43 of the lens case 40 being gradually reduced toward the tip end thereof. That is, the transverse dimension C of the space 45 - which receives the intraocular lens 10 placed on the lens top case 41 of the lens case 40 as shown in Figure 3D - is reduced to a transverse dimension D which corresponds to that measured at the tip end of the through hole 43 as shown in Figure 3B. This configuration enables the intraocular lens 10 to enter a deformed state from a non-deformed state while being moved through the through hole 43 by means of the push rod 30. At the tip end, the through hole 43 has an asymmetrical shape with respect to the vertical direction. Further, a rail 46 projecting toward the centre of the through hole 43 is formed such that the rail 46 extends in the direction of movement of the intraocular lens 10. This configuration enables the intraocular lens 10 to be formed into an intended shape.

Moreover, data display means 50 is provided on the outer surface of the device body 21 in order to visually display data, such as optical characteristics, of the intraocular lens 10.

Specifically, a sterilized, printed label serving as the display means 50 is bonded to the outer surface of the device body 21 in the vicinity of the attachment portion 23 to which the lens case 40 is attached. Data 51, such as optical characteristics, of the intraocular lens 10 placed within the lens case 40 are printed on the label.

When the intraocular lens insertion device according the present embodiment having the above-described configuration is manufactured at a plant, an intraocular lens is loaded into the insertion device in order to obtain a lens-containing insertion device; and a printed label carrying data of the loaded intraocular lens, such as performance and specifications of the intraocular lens, is bonded to the device body of the insertion device.

In the intraocular lens insertion device of the present embodiment, the data display means 50 for displaying data 51, such as optical characteristics, of the intraocular lens 10 placed within the lens case 40 of the insertion device 20 is provided at a visible position on the outer surface of the device body 21 of the intraocular lens insertion device 20. Therefore, even when the intraocular lens insertion device 20 is shipped from a plant in the form of a lens-containing insertion device onto which a new intraocular lens 10 has been loaded, the data 51, such as optical characteristics, of the intraocular lens 10 within the insertion device 20 can be confirmed with ease immediately before an operation, to thereby prevent an improper operation such as use of a wrong intraocular lens.

Examples of the data 51 regarding the intraocular lens 10 include the name of a manufacturer of the lens, the model name of the lens, the power of the lens, an effective sterilization period, information regarding a manner of use (fixing position), information regarding use of the lens such as matters to be attended to, the manufacturing date of the lens, a serial number, and a model number.

The data display means 50 may assume any of the following forms:
(1) a set of character data;
(2) a change in color provided at a predetermined location on the device body 21 of the intraocular lens insertion device 20;
(3) a string of characters engraved, by use of a laser, at an externally visible location on the outer surface of the device body 21 of the intraocular lens insertion device 20;
(4) a printed decal which carries character data and is visibly attached to the outer surface of the device body 21 of the intraocular lens insertion device 20; and
(5) a string of characters or marks formed at an externally visible position on the device body 21 of the intraocular lens insertion device 20 during a moulding process for moulding the device body 21.

Moreover, the lens data can be displayed not only by means of characters, but also by means of lines, dots, colours, or code.

In the above-described embodiment, the intraocular lens insertion device 20 has been described as being shipped from a plant in the form of a lens-containing insertion device loaded with an intraocular lens 10. However, the present invention is not limited thereto, and can be applied to the case in which a conventional insertion device is used; i.e., the case in which an intraocular lens 10 is removed from a lens storage container 18 and placed on an insertion device 20 immediately before an operation. In this case, a decal on which data of the intraocular lens 10 is printed is stored in the lens storage container 18 together with the intraocular lens 10; and simultaneously with loading of the intraocular lens 10 onto the insertion device 20, the decal is attached to a visible position of the outer surface of the device body. This enables an operating surgeon to confirm optical characteristics of an intraocular lens immediately before insertion thereof, even when the operating surgeon does not load the intraocular lens onto an intraocular lens insertion device.

In addition, even in the case in which insertion of an intraocular lens is performed by use of an operation tool, such as tweezers (not shown), other than the insertion tool 20 having a tapered insertion tube, data regarding the intraocular lens can be confirmed through employment of a lens storage container 18 which contains a decal on which data of the intraocular lens 10 have been printed or an insertion device on which data of the intraocular lens 10 have been imprinted.

Obviously, numerous modifications and variations of the present invention are possible in light of the above teachings. It is therefore to be understood that within the scope of the appended claims, the present invention may be practiced otherwise than as specifically described herein.

In this specification, the verb "comprise" has its normal dictionary meaning, to denote non-exclusive inclusion. That is, use of the word "comprise" (or any of its derivatives) to include one feature or more, does not exclude the possibility of also including further features.

## Claims

1. An intraocular lens insertion device (20) for inserting an intraocular lens (10) into an eye, **characterised in that** the device comprises data display means (50) which displays data (51) regarding a lens (10) loaded into the intraocular lens insertion device (20), the data display means (50) being provided at a predetermined visible position on an outer surface of the intraocular lens insertion device (20) so as to enable the data (51) regarding the loaded lens (10) to be checked from the outside of the intraocular lens insertion device (20).

2. An intraocular lens insertion device (20) according to claim 1, wherein the data display means (50) is a set of character data (51).

3. An intraocular lens insertion device (20) according to claim 1, wherein the data display means (50) is a change in colour provided at a predetermined location on a device body of the intraocular lens insertion device (20).

4. An intraocular lens insertion device (20) according to claim 1 or 2, wherein the data display means (50) is a string of characters engraved, by use of a laser, at an externally visible location on an outer surface of a device body of the intraocular lens insertion device (20).

5. An intraocular lens insertion device (20) according to claim 1 or 2, wherein the data display means (50) is a printed decal which carries character data and is visibly attached to an outer surface of a device body of the intraocular lens insertion device (20).

6. An intraocular lens insertion device (20) according to claim 1 or 2, wherein the data display means (50) is a string of characters moulded at an externally visible position on a device body of the intraocular lens insertion device (20).

7. An intraocular lens insertion device (20) according to claim 1 or 2, wherein the data display means (50) is a string of characters printed directly on an outer surface of a device body of the intraocular lens insertion device (20).

8. A method of preparing an intraocular lens (10) for insertion, including the step of loading the lens (10) into an intraocular lens insertion device (20), and
**characterised by** the step of providing on the insertion device (20) data display means (50) displaying data (51) regarding the loaded lens (10) such that said data (51) is visible and capable of being checked from the outside of the insertion device (20), prior to inserting the intraocular lens (10).

9. A method according to claim 8, wherein the intraocular lens insertion device (20) is in accordance with any of claims 1 to 7.

## Patentansprüche

1. Augenlinsen-Einführungsvorrichtung (20) zum Einführen einer Augenlinse (10) in ein Auge, **dadurch gekennzeichnet, daß** die Vorrichtung ein Datenanzeigemittel (50) umfaßt, welches Daten (51) hinsichtlich einer Linse (10), die in der Augenlinsen-Einführungsvorrichtung (20) aufgenommen ist, anzeigt, wobei das Datenanzeigemittel (50) an einer vorbestimmten sichtbaren Position an einer Außenoberfläche der Augenlinsen-Einführungsvorrichtung (20) bereitgestellt ist, um zu ermöglichen, daß die Daten (51) hinsichtlich der aufgenommenen Linse (10) von dem Äußeren der Augenlinsen-Einführungsvorrichtung (20) überprüfbar sind.

2. Augenlinsen-Einführungsvorrichtung (20) nach Anspruch 1, wobei das Datenanzeigemittel (50) ein Satz von Zeichendaten (51) ist.

3. Augenlinsen-Einführungsvorrichtung (20) nach Anspruch 1, wobei das Datenanzeigemittel (50) eine Farbänderung ist, die an einem vorbestimmten Ort auf einem Vorrichtungskörper der Augenlinsen-Einführungsvorrichtung (20) bereitgestellt wird.

4. Augenlinsen-Einführungsvorrichtung (20) nach Anspruch 1 oder 2, wobei das Datenanzeigemittel (50) eine Folge von Zeichen ist, die mittels eines Lasers an einer äußeren sichtbaren Position an einer Außenoberfläche eines Vorrichtungskörpers der Augenlinsen-Einführungsvorrichtung (20) eingraviert wurde.

5. Augenlinsen-Einführungsvorrichtung (20) nach Anspruch 1 oder 2, wobei das Datenanzeigemittel (50) ein gedrucktes Abziehbild ist, das Zeichendaten trägt und das sichtbar an einer Außenoberfläche eines Vorrichtungskörpers der Augenlinsen-Einführungsvorrichtung (20) angeordnet ist.

6. Augenlinsen-Einführungsvorrichtung (20) nach Anspruch 1 oder 2, wobei das Datenanzeigemittel (50) eine Folge von Zeichen ist, die an einer äußeren sichtbaren Position an einem Vorrichtungskörper der Augenlinsen-Einführungsvorrichtung (20) geformt ist.

7. Augenlinsen-Einführungsvorrichtung (20) nach Anspruch 1 oder 2, wobei das Datenanzeigemittel (50) eine Folge von Zeichen ist, die direkt an einer Außenoberfläche eines Vorrichtungskörpers der Augenlinsen-Einführungsvorrichtung (20) gedruckt wurde.

8. Verfahren zum Herstellen einer Augenlinse (10) zum Einführen, mit dem Schritt des Aufnehmens der Linse (10) in eine Augenlinsen-Einführungsvorrichtung (20) und **gekennzeichnet durch** den Schritt des Bereitstellens eines Datenanzeigemittel (50) an der Einführungsvorrichtung (20), das Daten (51) hinsichtlich der aufgenommenen Linse (10) anzeigt, so daß die Daten (51) sichtbar und geeignet sind, von dem Äußeren der Einführungsvorrichtung (20) geprüft zu werden, vor dem Einführen der Augenlinse (10).

9. Verfahren nach Anspruch 8, bei dem die Augenlinsen-Einführungsvorrichtung (20) in Übereinstimmung mit einem der Ansprüche 1 bis 7 ist.

## Revendications

1. Dispositif (20) anti-intraoculaire pour l'insertion d'une lentille intraoculaire (10) dans un oeil, **caractérisé en ce qu'**il comporte des moyens d'affichage de données (50) qui affichent des données (51) concernant une lentille (10) chargée dans un dispositif (20) d'insertion de lentille intraoculaire, les moyens d'affichage de données (50) étant placés en une position très déterminée visible sur une surface extérieure du dispositif d'insertion de lentille intraoculaire (20) afin de permettre aux données (21) concernant la lentille chargée (10), d'être vérifiées depuis l'extérieur du dispositif (20) d'insertion de lentille intraoculaire.

2. Dispositif d'insertion de lentille intraoculaire (20) selon la revendication 1, **caractérisé en ce que** les moyens d'affichage de données sont réglés à une donnée de caractère (51).

3. Dispositif d'insertion de lentille intraoculaire (20) selon la revendication 1, **caractérisé en ce que** les moyens (50) d'affichage de données sont un changement de couleur, disposés en un endroit donné sur un corps de dispositif du dispositif (20) d'insertion intraoculaire de lentille.

4. Dispositif d'insertion de lentille intraoculaire (20) selon la revendication 1 ou 2, **caractérisé en ce que** les moyens d'affichage (50) de données sont une chaîne de caractères gravés, à l'aide d'un laser, en une position visible externe, sur une surface externe d'un corps de dispositif du dispositif d'insertion de lentille intraoculaire (20).

5. Dispositif d'insertion de lentille intraoculaire (20) selon la revendication 1 ou 2, **caractérisé en ce que** les moyens d'affichage de données (50) sont un élément décalqué imprimé qui porte des données de caractère et qui est attaché de manière visible à une surface extérieure d'un corps de dispositif du dispositif (20) d'insertion de lentille intraoculaire.

6. Dispositif d'insertion de lentille intraoculaire (20) selon la revendication 1 ou 2, **caractérisé en ce que** les moyens d'affichage de moyens de données (50) sont une chaîne de caractères moulés en une position externe visible d'un corps de dispositif du dispositif d'insertion de lentille intraoculaire (20).

7. Dispositif d'insertion de lentille intraoculaire (20) selon la revendication 1 ou 2, **caractérisé en ce** les moyens d'affichage de données (50) sont une chaîne de caractères imprimés directement sur une surface extérieure d'un corps de dispositif d'insertion de lentille intraoculaire (20).

8. Procédé pour la préparation d'une lentille (10) intraoculaire en vue de son insertion, incluant l'étape de charger la lentille (10) dans un dispositif d'insertion de lentille intraoculaire (20), **caractérisé par** l'étape de fournir sur le dispositif d'insertion (20) des moyens d'affichage de données (50) affichant des données (51) concernant la lentille chargée, de façon que lesdites données (51) soient visibles et capables d'être vérifiées depuis l'extérieur du dispositif d'insertion (20), avant l'insertion de la lentille intraoculaire (10).

9. Procédé selon la revendication 8, **caractérisé en ce que** le dispositif (20) d'insertion de lentille intraoculaire est conforme à l'une des revendications 1 à 7.
